(19) Europäisches Patentamt
European Patent Office
Office européen des brevets

(11) Veröffentlichungsnummer: **0 435 219 A2**

# (12) EUROPÄISCHE PATENTANMELDUNG

(21) Anmeldenummer: **90125270.0**

(22) Anmeldetag: **21.12.90**

(51) Int. Cl.5: **C07C 217/28**, C07C 229/12, C07C 309/14, C11D 1/00

(30) Priorität: **28.12.89 DE 3943128**

(43) Veröffentlichungstag der Anmeldung: **03.07.91 Patentblatt 91/27**

(84) Benannte Vertragsstaaten: **BE CH DE ES FR GB IT LI NL**

(71) Anmelder: **HOECHST AKTIENGESELLSCHAFT**
**Postfach 80 03 20**
**W-6230 Frankfurt am Main 80(DE)**

(72) Erfinder: **Knaup, Wolfgang, Dr.**
**Kanalstrasse 3**
**W-8269 Burgkirchen(DE)**
Erfinder: **Wehowsky, Frank, Dr.**
**Am Apfelgärtchen 11**
**W-6272 Niedernhausen(DE)**
Erfinder: **Schmitt, Norbert**
**Kastenstrasse 6**
**W-8269 Burgkirchen(DE)**

## (54) Oberflächenaktive Verbindungen mit einer Perfluoralkylgruppe und einem Stickstoff enthaltenden aliphatischen Rest, Verfahren zu ihrer Herstellung und ihre Verwendung.

(57) Die neuen Verbindungen entsprechen der nachstehenden Formel

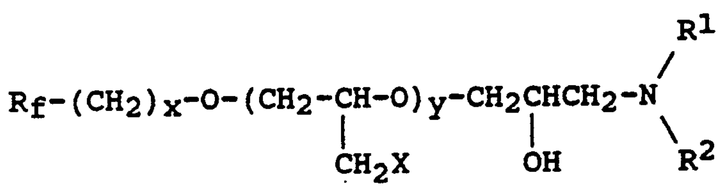

worin bedeuten:

| | |
|---|---|
| $R_f$ | einen Perfluoralkylrest mit 4 bis 20 C-Atomen, |
| x | 1 bis 4, |
| y | 1 bis 10, |
| X | Br, Cl oder I, und |
| $R^1$ und $R^2$ | Wasserstoff oder einen $C_1$ bis $C_4$-Alkylrest oder einen wasserlöslich machenden aliphatischen Rest, mit der Maßgabe, daß mindestens einer der beiden Substituenten $R^1$ und $R^2$ ein wasserlöslich machender aliphatischer Rest ist. |

Die beschriebenen oberflächenaktiven Verbindungen werden hergestellt durch Reaktion eines entsprechenden Epoxids mit einem entsprechenden Amin. Sie werden insbesondere zur Bereitung von Schaummitteln verwendet.

EP 0 435 219 A2

# OBERFLÄCHENAKTIVE VERBINDUNGEN MIT EINER PERFLUORALKYLGRUPPE UND EINEM STICKSTOFF ENTHALTENDEN ALIPHATISCHEN REST, VERFAHREN ZU IHRER HERSTELLUNG UND IHRE VERWENDUNG

Die Erfindung betrifft oberflächenaktive Verbindungen mit einer Perfluoralkylgruppe und einem Stickstoff enthaltenden aliphatischen Rest, ein Verfahren zur Herstellung dieser Verbindungen und ihre Verwendung.

Für bestimmte Zwecke, zum Beispiel zur Bereitung von Schaummitteln, werden chemische Verbindungen benötigt, die eine wesentliche Erniedrigung der Oberflächenspannung des Wassers oder wäßriger Systeme und zusätzlich dazu eine starke Schaumbildung bewirken. Aus dem Stand der Technik ergibt sich, daß zumindest eine der beiden Eigenschaften solche Verbindungen aufweisen, die eine Perfluoralkylgruppe und einen Stickstoff enthaltenden aliphatischen Rest im Molekül besitzen.

So werden in der US-Patentschrift 3 836 522 oberflächenaktive Perfluoralkylamidamin-Verbindungen beschrieben. Im Vergleich zu analogen Fluortensiden sind sie besser wasserlöslich und setzen die Oberflächenspannung von wäßrigen Systemen bei gleicher Einsatzmenge in einem höheren Ausmaß herab. Bezüglich Schaumbildung lassen sie jedoch zu wünschen übrig.

Zu erwähnen ist hier auch die deutsche Offenlegungsschrift DE 37 24 198-A1, in der Perfluoralkyl und Epihalogenhydrin enthaltende Epoxide beschrieben sind. Die beschriebenen Epoxide (vergleiche Ansprüche 7 und 8) entsprechen der Formel

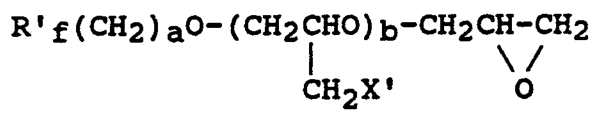

worin bedeuten:

- $R'_f$ einen Perfluoralkylrest mit 4 bis 20 C-Atomen, vorzugsweise 6 bis 16 C-Atomen,
- a 1 bis 4, vorzugsweise 2, b 1 bis 20, vorzugsweise 1 bis 10, und
- X' Br, Cl oder I, vorzugsweise Br oder Cl.

Bezüglich der Eigenschaften dieser Epoxide wird gesagt, daß sie leicht polymerisierbar sind und daher ein vorteilhaftes Monomeres zur Herstellung von fluorhaltigen Epoxidharzen darstellen, daß sie ferner eine hohe Verträglichkeit mit nicht-fluorierten Epoxiden besitzen und Textilien und Leder eine gute Oleophobie und Hydrophobie verleihen.

Ausgehend vom Stand der Technik, wonach Verbindungen mit einer Perfluoralkylgruppe und einem Stickstoff enthaltenden aliphatischen Rest vorteilhafte oberflächenaktive Eigenschaften aufweisen, liegt der Erfindung die Aufgabe zugrunde, nach weiteren wirksamen Verbindungen dieser Art zu suchen. Die neuen Verbindungen sollen gut wasserlöslich sein und auch schon in einer geringen Menge eine beträchtliche Erniedrigung der Oberflächenspannung des Wassers oder wäßriger Systeme bewirken. Darüber hinaus sollen sie auch ein hohes Schaumbildungsvermögen aufweisen. Mit den neuen Verbindungen soll also in Wasser oder wäßrigen Systemen sowohl Oberflächenspannungserniedrigung als auch Schaumbildung erreicht werden.

Die erfindungsgemäßen oberflächenaktiven Verbindungen sind gekennzeichnet durch die nachstehende allgemeine Formel 1

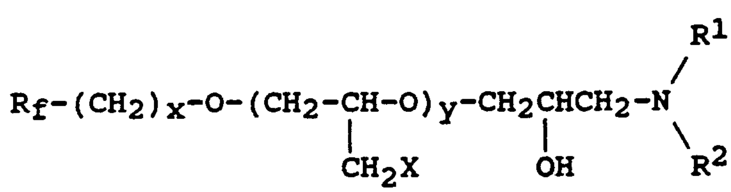 (1)

worin bedeuten:

- $R_f$ einen Perfluoralkylrest mit 4 bis 20 C-Atomen, vorzugsweise 6 bis 16 C-Atomen,
- x 1 bis 4, vorzugsweise 2,
- y 1 bis 10, vorzugsweise 1 bis 5,
- X Br, Cl oder I, vorzugsweise Cl, und
- $R^1$ und $R^2$ Wasserstoff oder einen $C_1$ bis $C_4$-Alkylrest oder einen wasserlöslich machenden aliphatischen Rest, mit der Maßgabe, daß mindestens einer der beiden Substituenten $R^1$ und $R^2$ ein wasserlöslich machender aliphatischer Rest ist.

Die Erfindung beruht auf der überraschenden Feststellung, daß aus der Kombination der in der genannten deutschen Offenlegungsschrift DE 37 24 198-A1 beschriebenen Perfluoralkyl und Epihalogenhydrin enthaltenden Epoxide, das heißt Epoxide der nachstehenden Formel 2

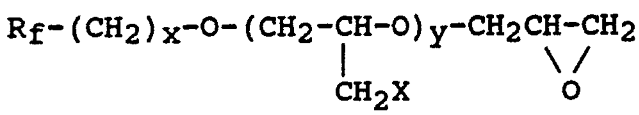 (2)

worin $R_f$, x, y und X die genannten Bedeutungen haben, mit bestimmten aliphatischen Aminverbindungen Produkte resultieren, welche die gestellte Aufgabe in einem besonders hohen Ausmaß erfüllen.

Die Perfluoralkylgruppe $R_f$ kann gerade oder verzweigt, gesättigt oder ungesättigt (mit vorzugsweise 1 bis 3 Doppelbindungen) sein, wobei gesättigt bevorzugt ist. Im Falle einer verzweigten Perfluoralkylgruppe ist die endverzweigte bevorzugt. Beim Perfluoralkylrest handelt es sich häufig um ein Gemisch von Perfluoralkyl mit der genannten Anzahl von C-Atomen, nämlich 4 bis 20 C-Atomen, vorzugsweise 6 bis 16 C-Atomen.

$R^1$ und $R^2$ bedeuten einen wasserlöslich machenden aliphatischen Rest oder einen $C_1$ bis $C_4$-Alkylrest oder das Wasserstoffatom, wobei aber nur $R^1$ oder nur $R^2$ ein $C_1$ bis $C_4$-Alkylrest oder H ist. Am Stickstoffatom der Formel 1 soll also mindestens ein wasserlöslich machender aliphatischer Rest sein. Bezüglich des wasserlöslich machenden aliphatischen Restes können $R^1$ und $R^2$ gleich oder verschieden sein, sie sind vorzugsweise gleich. Die wasserlöslich machenden Reste sind vorzugsweise solche aus der Gruppe der Hydroxyalkylenreste, Alkylencarboxylatreste, Alkylensulfonatreste und der Reste der Formel

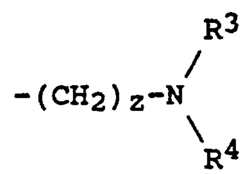

worin z 1 bis 10 ist, vorzugsweise 1 bis 5, und $R^3$ und $R^4$ die Bedeutungen von $R^1$ und $R^2$ haben. Besonders bevorzugte wasserlöslich machende aliphatische Reste sind solche aus der Gruppe der Hydroxyalkylenreste der Formel $-(CH_2)_m-OH$, Alkylencarboxylatreste der Formel $-(CH_2)_n-COOMe$, Alkylensulfonatreste der Formel $-(CH_2)_w-SO_3Me$ und der Reste der Formel

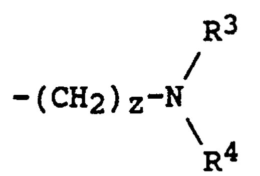

wobei $R^3$ und $R^4$ die Bedeutungen von $R^1$ und $R^2$ haben, vorzugsweise jeweils $-CH_2CH_2OH$ sind, m, n, w und z 1 bis 10 sind, vorzugsweise 1 bis 5, und Me H oder vorzugsweise ein Kation ist. Das Kation ist vorzugsweise ein Alkalimetallkation oder ein Ammonium- oder Organoammoniumkation.

Nachstehend werden einige beispielhafte Verbindungen der Formel 1 angegeben, wobei der in Formel 1 stehende Ausdruck

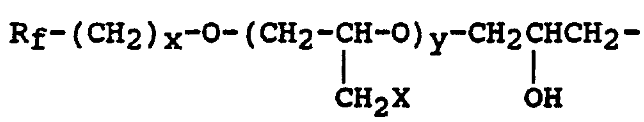

gleich A gesetzt wird und Me die genannten Bedeutungen hat:

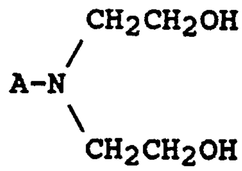

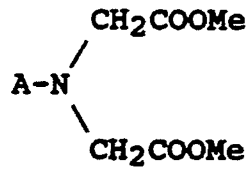

$A-NH-CH_2COOMe$

$A-N(CH_3)-CH_2COOMe$

$A-NH-(CH_2)_5-COOMe$

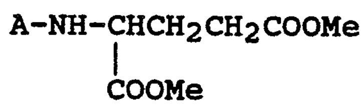

$A-NH-CH_2CH_2SO_3Me$

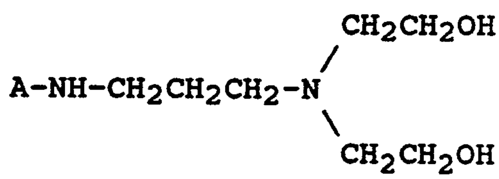

Die Herstellung der erfindungsgemäßen Verbindungen ergibt sich aus der allgemeinen Formel 1. Nach einem bevorzugten Verfahren erfolgt ihre Herstellung durch Umsetzung der obengenannten Epoxide der Formel 2

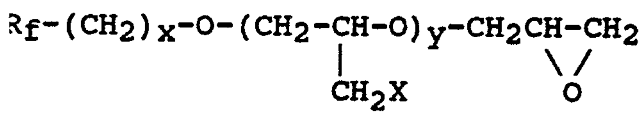
(2)

mit einem aliphatischen Amin der nachstehenden Formel 3

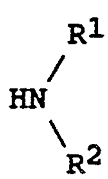
(3)

worin R¹ und R² die angegebenen Bedeutungen haben, im Molverhältnis von 1 : 1.

Die Epoxide der Formel 2 und ihre Herstellung sind in der genannten deutschen Offenlegungsschrift DE 37 24 198-A1, die hier mit einbezogen wird, ausführlich beschrieben. Es handelt sich um flüssige bis wachsartig feste, gelb bis braun gefärbte Produkte. Sie werden dadurch erhalten, daß ein Fluoralkohol-Epihalogenhydrin-Addukt der nachstehenden Formel

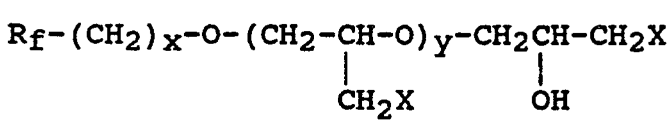

worin $R_f$, x, y und X die genannten Bedeutungen haben, zur Abspaltung von Halogenwasserstoff unter Cyclisierung der Halogenhydringruppe des Adduktes mit Alkalimetall- oder Erdalkalimetallhydroxiden in Form einer wäßrigen Lösung, in Gegenwart von Phasentransferkatalysatoren und bei einem pH-Wert von 9 bis 12 umgesetzt und die gebildete, angestrebte Epoxidverbindung aus der Reaktionsmischung isoliert wird. Die aliphatischen Amine der genannten Formel 3 gehören ebenfalls zum Stand der Technik. Es handelt sich, wie ohne weiteres ersichtlich ist, um wohlbekannte Produkte.

Die Herstellung der erfindungsgemäßen oberflächenaktiven Verbindungen erfolgt durch Umsetzung eines Epoxids der Formel 2 mit einem aliphatischen Amin der Formel 3 im Molverhältnis von 1 : 1. Die Umsetzungstemperatur liegt bei 40 bis 120 °C, vorzugsweise bei 50 bis 100 °C. Die Umsetzung kann in Schmelze, das heißt in Substanz, oder unter Verwendung eines Lösungsmittels durchgeführt werden. Bevorzugte Lösungsmittel sind die $C_1$ bis $C_4$-Alkanole. Die Reaktion zwischen der Aminverbindung und dem Epoxid verläuft quantitativ. Wenn die Umsetzung nicht in Substanz vorgenommen wird, kann die angestrebte Verbindung zum Beispiel dadurch isoliert werden (falls sie nicht ohnehin in Lösung gewünscht wird), daß das Lösungsmittel und gegebenenfalls vorhandenes Wasser destillativ abgetrennt werden. Die erfindungsgemäßen Verbindungen stellen bei Raumtemperatur flüssige bis wachsartige, weiß bis braun gefärbte Produkte dar.

Die erfindungsgemäßen Verbindungen weisen eine Reihe von Vorteilen auf. Sie sind gut wasserlöslich und bewirken in Wasser oder wäßrigen Systemen auch schon in einer geringen Menge nicht nur eine beträchtliche Oberflächenspannungserniedrigung, sondern auch eine starke Schaumbildung. Aufgrund dieser Eigenschaftskombination sind sie vielfach verwendbar, insbesondere als Schaummittel. Dies gilt insbesondere für jene erfindungsgemäßen Verbindungen, die als wasserlöslich machende Gruppe die genannten Alkylencarboxylatreste enthalten. Die Bereitung eines Schaummittels mit den erfindungsgemäßen Verbindungen erfolgt nach bekannten Methoden. Man bereitet eine wäßrige Lösung, die mindestens eine erfindungsgemäße Verbindung in einer wirksamen Menge enthält, das heißt in einer solchen Menge, daß die Lösung beim Rühren, Schütteln oder Verspritzen mit Hilfe von Luft oder einem anderen Treibmittel, die angestrebte Schaummenge erzeugt. Diese Menge, ausgedrückt in Gramm pro Liter Wasser, liegt bei 0,1 bis 10 g/l, vorzugsweise 0,5 bis 5 g/l, das sind 0,01 bis 1 Gew.-%, vorzugsweise 0,05 bis 0,5 Gew.-%, Gewichtsprozente bezogen auf das Gewicht der wäßrigen Lösung. Die erfindungsgemäßen Verbindungen können mit den üblichen Schaumhilfsmitteln kombiniert werden, zum Beispiel mit Proteinprodukten, wie Keratine, Albumine, Globuline, Samenmehl und dergleichen, gegebenenfalls modifiziert durch Hydrolyse und stabilisiert mit Salzen von mehrwertigen Metallen. Als Anwendungsgebiet für Schaummittel mit den erfindungsgemäßen Verbindungen sei das Löschen von Bränden von flüssigen Kohlenwasserstoffen, wie Fahrzeugbenzin, Flugbenzin und dergleichen, genannt. Dabei wird bekanntlich durch Verspritzen des Schaummittels eine dicke Schaumdecke über den gesamten brennenden Bereich ausgebreitet.

Die Erfindung wird nun an Beispielen noch näher erläutert.

Beispiel 1

In einem mit Rührer ausgestatteten Reaktionsgefäß wurden vorgelegt 68,4 g (0,651 mol) von dem Amin der Formel

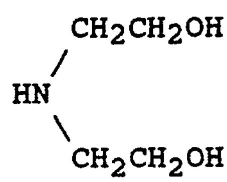

das ist Diethanolamin.

Das vorgelegte Produkt wurde auf 60 °C erhitzt, und bei dieser Temperatur wurden 360,0 g (0,651 mol) von dem Epoxid der Formel

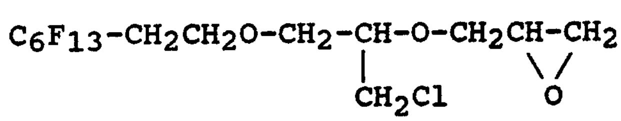

im Verlauf einer Stunde zugegeben. Nach Zugabe der Epoxidmenge wurde die Mischung noch weitere 6 Stunden bei 60 °C (unter Rühren) gehalten. Nach dieser Zeit hatten das Amin und das Epoxid vollständig zur erfindungsgemäßen Verbindung der Formel

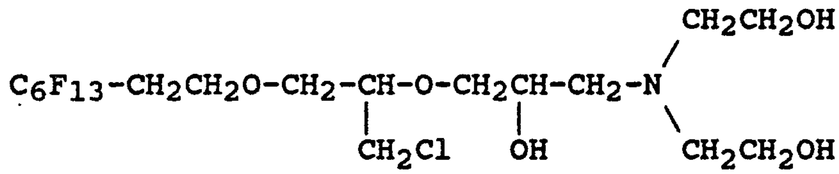

reagiert. Es lag ein bei Raumtemperatur mehr oder weniger wachsartiges, gelb gefärbtes Produkt vor.

Beispiel 2

In einem mit Rührer ausgestatteten Reaktionsgefäß wurden 18,2 g (0,136 mol) Iminodiessigsäure, 19,8 g Isopropanol und 10,9 g (0,272 mol) NaOH in Form einer 18gew.%igen wäßrigen Lösung vorgelegt. Die Mischung wurde auf 60 °C erhitzt, und bei dieser Temperatur wurden 70,0 g (0,136 mol) Epoxid der Formel

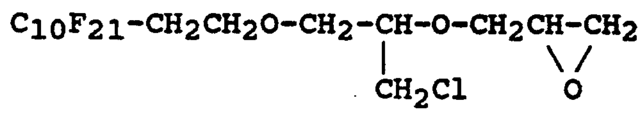

im Verlauf von 4 Stunden zugegeben. Nach Zugabe der Epoxidmenge wurde die Mischung noch weitere 4 Stunden bei 65 °C gehalten. Nach dieser Zeit hatten die Aminverbindung und das Epoxid vollständig zur erfindungsgemäßen Verbindung der Formel

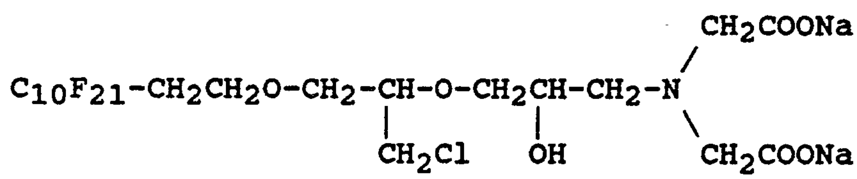

reagiert. Die erfindungsgemäße Verbindung, ein wachsartiges, gelb gefärbtes Produkt, lag in Form einer Dispersion in dem genannten Lösungsmittel vor (der Perfluoralkylrest $C_{10}F_{21}$ steht für das Perfluoralkylgemisch $C_8F_{17}$ bis $C_{16}F_{33}$).

Beispiel 3

In einem mit Rührer ausgestatteten Reaktionsgefäß wurden 21,6 g (0,165 mol) ε-Aminocapronsäure, 100 g Methanol und 6,6 g (0,165 mol) NaOH in Form einer 40gew.%igen wäßrigen Lösung vorgelegt. Die Mischung wurde auf 65 °C erhitzt, und bei dieser Temperatur wurden 91,0 g (0,165 mol) Epoxid vom Beispiel 1 im Verlauf einer Stunde zugegeben. Nach Zugabe der Epoxidmenge wurde die Mischung noch weitere 6 Stunden bei 65 °C gehalten. Nach dieser Zeit hatten die Aminverbindung und das Epoxid vollständig zur erfindungsgemäßen Verbindung der Formel

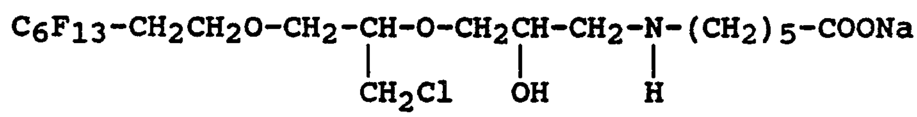

reagiert. Nach dem destillativen Abtrennen des anwesenden Wassers und des Methanols lag die erfindungsgemäße Verbindung als wachsartiges, gelb gefärbtes Produkt vor.

Beispiel 4

**Ansatz:**

27,8 g (0,189 mol) Glutaminsäure

200 ml Methanol

15,1 g (0,378 mol) NaOH in Form einer 40gew.%igen wäßrigen Lösung

100,0 g (0,189 mol) Epoxid vom Beispiel 1.

Durchführung: wie in Beispiel 3.

Die erfindungsgemäße Verbindung, ein wachsartiges, gelb gefärbtes Produkt, entspricht der nachstehenden Formel

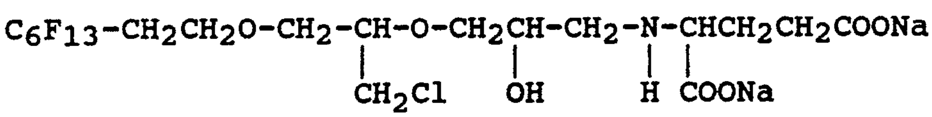

Beispiel 5

Ansatz:

77,9 g (0,48 mol) Aminverbindung der Formel

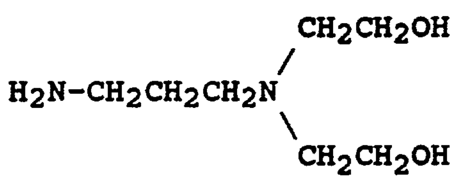

301,9 g (0,48 mol) Epoxid vom Beispiel 2.

Durchführung: wie im Beispiel 1.

Die erfindungsgemäße Verbindung, ein wachsartiges, gelb gefärbtes Produkt, entspricht der nachstehenden Formel

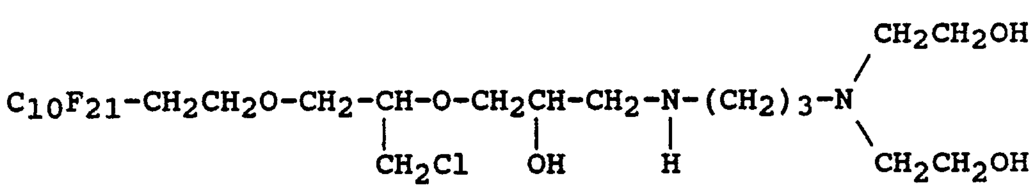

Beispiel 6

Beispiel 2 wurde wiederholt, mit dem Unterschied, daß 107,2 g (0,136 mol) Epoxid der Formel

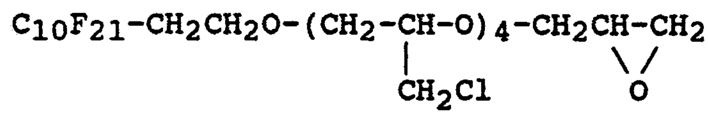

im Verlauf von 4 Stunden zugegeben wurden. Die erhaltene erfindungsgemäße Verbindung entspricht bezüglich Aussehen jener des Beispiels 2 und hat die analoge Formel.

Die erfindungsgemäßen Verbindungen der Beispiele 1 bis 6 und eine Verbindung des Standes der Technik, nämlich die Verbindung des Beispiels 1 der eingangs genannten US-Patentschrift 3 836 552 mit der Formel

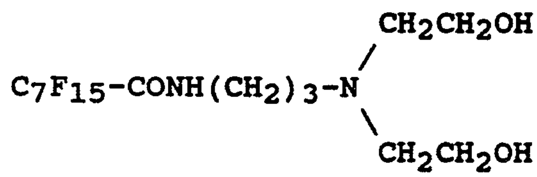

wurden bezüglich Oberflächenspannungserniedrigung und Schaumbildung getestet.

Die Oberflächenspannung wurde nach DIN 53 914 bestimmt. Es wurden 0,1 g und 1 g von jeder Verbindung in jeweils einem Liter vollentsalztem Wasser gelöst. Von diesen Lösungen wurde die Oberflächenspannung bei 20 °C mit einem üblichen Tensiometer nach der Ringabreißmethode in mN/m gemessen.

Die Schaumbildung wurde nach DIN 53 902 bestimmt. Es wurden 0,5 g und 2 g von jeder Verbindung in jeweils einem Liter vollentsalztem Wasser gelöst. Von diesen Lösungen wurden 200 ml in den 1000-ml-Meßzylinder gegeben und bei 25 °C mit der gelochten Schlagscheibe dreißigmal geschlagen. Die entstandene Schaumhöhe im Meßzylinder, ausgedrückt in Milliliter, stellt den Schaumwert dar.

Die Ergebnisse sind in der nachstehenden Tabelle zusammengefaßt.

Die erfindungsgemäßen Verbindungen besitzen einen beträchtlich höheren Schaumwert, das heißt ein wesentlich stärkeres Schaumbildungsvermögen, als die Verbindungen des Standes der Technik. Bezüglich Erniedrigung der Oberflächenspannung weist die Verbindung des Standes der Technik einen guten Wert auf. Aber auch die Werte der erfindungsgemäßen Verbindungen sind noch gut, zumal die Oberflächenspannung des Wassers (75 mN/m bei 20 °C) um mehr als 70 % herabgesetzt wird. Im Hinblick auf die Kombination der beiden Eigenschaften, nämlich Oberflächenspannungserniedrigung und Schaumbildung, sind also die erfindungsgemäßen Verbindungen dem Stand der Technik in einem unerwarteten Ausmaß überlegen.

Tabelle

| Verbindungen der Beispiele 1 bis 6 | Oberflächenspannung (mN/m) | | Schaumwert (ml) | |
|---|---|---|---|---|
| | 0,1 g/l | 1 g/l | 0,5 g/l | 2 g/l |
| 1 | 21 | 18 | 70 | 150 |
| 2 | 23 | 17 | 500 | 670 |
| 3 | 24 | 20 | 260 | 570 |
| 4 | 23 | 19 | 380 | 660 |
| 5 | 25 | 26 | 100 | 270 |
| 6 | 25 | 22 | 480 | 640 |
| Vergleichsverbindung | 21 | 18 | 50 | 100 |

## Ansprüche

1. Oberflächenaktive Verbindungen mit einer Perfluoralkylgruppe und einem Stickstoff enthaltenden aliphatischen Rest, gekennzeichnet durch die nachstehende Formel 1

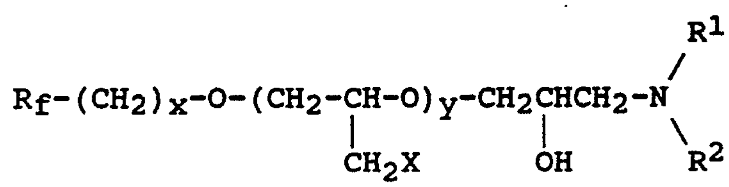

(1)

worin bedeuten:

$R_f$ einen Perfluoralkylrest mit 4 bis 20 C-Atomen,
x 1 bis 4,
y 1 bis 10,
X Br, Cl oder I, und
$R^1$ und $R^2$ Wasserstoff oder einen $C_1$ bis $C_4$-Alkylrest oder einen wasserlöslich machenden aliphatischen Rest, mit der Maßgabe, daß mindestens einer der beiden Substituenten $R^1$ und $R^2$ ein wasserlöslich machender aliphatischer Rest ist.

2. Verbindungen nach Anspruch 1, wobei bedeuten:

$R_f$ einen Perfluoralkylrest mit 6 bis 16 C-Atomen,
x 2,
y 1 bis 5,
X Cl, und
$R^1$ und $R^2$ Wasserstoff oder einen $C_1$ bis $C_4$-Alkylrest oder einen wasserlöslich machenden aliphatischen Rest aus der Gruppe der Hydroxyalkylenreste, Alkylencarboxylatreste, Alkylensulfonatreste und der Reste der Formel

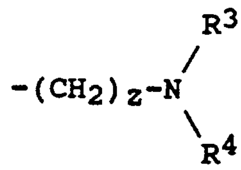

wobei

z 1 bis 10 ist und $R^3$ und $R^4$ die Bedeutungen von $R^1$ und $R^2$ haben.

3. Verbindungen nach Anspruch 1, wobei bedeuten:

$R_f$ einen Perfluoralkylrest mit 6 bis 16 C-Atomen,

x 2,

y 1 bis 5,

X Cl, und

$R^1$ und $R^2$ Wasserstoff oder einen $C_1$ bis $C_4$-Alkylrest oder einen wasserlöslich machenden aliphatischen Rest aus der Gruppe der Hydroxyalkylenreste der Formel $-(CH_2)_m-OH$, Alkylencarboxylatreste der Formel $-(CH_2)_n-COOMe$, Alkylensulfonatreste der Formel $-(CH_2)_w-SO_3Me$ und der Reste der Formel

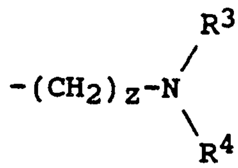

wobei

$R^3$ und $R^4$ die Bedeutungen von $R^1$ und $R^2$ haben, m, n, w und z 1 bis 10 sind und Me H oder ein Kation ist.

4. Verbindungen nach Anspruch 1, wobei bedeuten:

$R_f$ einen Perfluoralkylrest mit 6 bis 16 C-Atomen,

x 2,

y 1 bis 5,

X Cl, und

$R^1$ und $R^2$ Wasserstoff oder einen $C_1$ bis $C_4$-Alkylrest oder einen wasserlöslich machenden aliphatischen Rest aus der Gruppe der Hydroxyalkylenreste der Formel

$-(CH_2)_m-OH$, Alkylencarboxylatreste der Formel

$-(CH_2)_n-COOMe$, Alkylensulfonatreste der Formel

$-(CH_2)_w-SO_3Me$ und der Reste der Formel

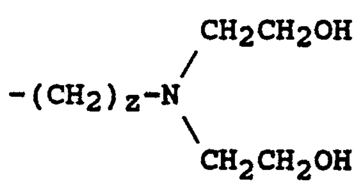

wobei

m, n, w und z 1 bis 5 sind und Me H oder ein Kation ist.

5. Verfahren zur Herstellung der Verbindungen nach Anspruch 1, dadurch gekennzeichnet, daß man ein Epoxid der Formel 2

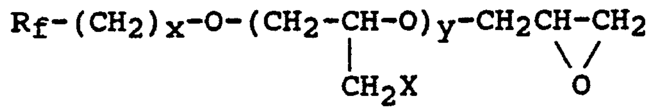

(2)

worin

$R_f$ , x, y und X die genannten Bedeutungen haben, mit einer aliphatischen Aminverbindung der Formel 3

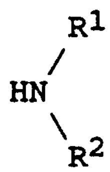

(3)

worin

$R^1$ und $R^2$ die genannten Bedeutungen haben, im Molverhältnis von 1 : 1 umsetzt.

**6.** Verwendung der Verbindungen nach Anspruch 1 zur Bereitung von Schaummitteln.